Europäisches Patentamt

European Patent Office

Office européen des brevets

(19)

(11) Veröffentlichungsnummer: **0 222 853
B1**

(12) **EUROPÄISCHE PATENTSCHRIFT**

(45) Veröffentlichungstag der Patentschrift: **08.08.90**

(51) Int. Cl.⁵: **A 61 F 2/30, A 61 L 27/00**

(21) Anmeldenummer: **86903313.4**

(22) Anmeldetag: **07.05.86**

(86) Internationale Anmeldenummer:
**PCT/EP86/00268**

(87) Internationale Veröffentlichungsnummer:
**WO 86/06617 20.11.86 Gazette 86/25**

(54) **IMPLANTATKÖRPER MIT BESCHICHTUNG.**

(30) Priorität: **07.05.85 DE 3516411**

(43) Veröffentlichungstag der Anmeldung:
**27.05.87 Patentblatt 87/22**

(45) Bekanntmachung des Hinweises auf die
Patenterteilung:
**08.08.90 Patentblatt 90/32**

(84) Benannte Vertragsstaaten:
**AT CH DE FR GB IT LI NL SE**

(56) Entgegenhaltungen:
**EP-A-0 006 544
EP-A-0 023 608
DE-A-2 313 678
DE-A-2 840 064
FR-A-2 336 913
FR-A-2 374 020
US-A-3 605 123**

(73) Patentinhaber: **Plasmainvent AG
Im Oberleh 2
CH-6300 Zug (CH)**

(72) Erfinder: **GRUNER, Heiko
Kehlenstrasse 1014
CH-5712 Beinwil am See (CH)**

(74) Vertreter: **Lehn, Werner, Dipl.-Ing. et al
Hoffmann, Eitle & Partner Patentanwälte
Arabellastrasse 4
D-8000 München 81 (DE)**

Courier Press, Leamington Spa, England.

**Beschreibung**

Die Erfindung bezieht sich auf einen Implantatkörper mit poriger, biokompatibler Beschichtung, der z.B. nach komplizierten Knochenbrüchen oder Knochenkrebs als Fremdteil in den menschlichen Körper eingepflanzt wird und dort die Funktion von Knochenersatz übernimmt.

Durch die DE—A—2 313 678 ist beispielsweise eine derartige Beschichtung eines Implantatkörpers aus Ti-Legierungen bekannt geworden, die aus Ti, $TiO_2$ oder $Al_2O_3$ besteht und an bestimmten Stellen des Implantatkörpers in einer einzigen Schicht z.B. durch Flammspritzen aufgebracht ist.

Durch die EP—A—0006544 ist eine Beschichtung eines Implantatkörpers aus reinem Metall bekannt geworden, die vier gleich ausgebildete und dicke Lagen aufweist. Die innerste Lage ist dort eine reinmetallische, poröse Haftshicht, die weiteren Lagen enthalten Calciumphosphat-Partikel mit unterschiedlichem Volumenteil von innen nach außen ansteigend.

In der Bundesrepublik Deutschland z.B. werden pro Jahr allein über 50 000 künstliche Hüftgelenke von der orthopädischen Chirurgie verarbeitet. Dabei kommen im wesentlichen zwei Techniken bei der Verbindung des Implantates mit den Humanknocken zur Anwendung. Mit Hilfe von Knochenzementen, meistens eine Mischung eines Pulvers und einer Flüssigkeit zu einem Brei verrührt, welcher durch Polymerisation beider Komponenten nach kurzer Zeit aushärtet, wird bei der älteren Verbindungstechnik das Implantat unmittelbar bei der Operation in eingesetzten Knochengewebe verankert. Allerdings förderte die nach einer bestimmten Gebrauchsdauer meist auftretende Implantatlockerung die Entwicklung der zweiten Verbindungstechnik, bei welcher die Implantate, durch geeignete Materialwahl und Oberflächengestaltung begünstigt, direkt vom Knochengewebe umwachsen werden. Bei diesen zementlosen Endprothesen konzentrierte sich lange Zeit das chirurgische Interesse auf die makroskopische Formgebung und auf die mechanische Langzeitfestigkeit des Implantatmaterials. Es wurden unterschiedlichste Materialien biomechanisch optimiert, Implantate aus Speziellegierungen, Kunststoffen oder Keramiken hergestellt und zumindest im Tierexperiment getestet.

Mehr und mehr setzte sich aber die Erkenntnis durch, daß die Funktiontüchtigkeit eines Implantates mehr von dem chemischen und funktionellen Zusammenspiel einer Oberfläche mit seiner biologischen Umgebung abhängig ist. Dafür verantwortlich ist die mikromorphologische Oberflächenbeschaffenheit. Sie beeinflußt die Wechselwirkung zwischen eingesetztem Fremdkörper und dem Knochen bzw. dem Periost und entscheidet damit, ob ein dauerhaftes Verwachsen eintritt oder eine oft entzündlich verlaufende Abwehrreaktion sogar die Wiederentfernung notwendig macht.

Auch die Oberflächenmorphologie betreffend geht heute die moderne Implantattechnik zwei Wege. Auf der einen Seite werden Implantate aus körper- oder knochenkompatiblen Materialien in einer speziellen Oberflächenstruktur hergestellt, mit dem Ziel, das Verwachsen günstig zu beeinflussen. So wurde z.B. bei metallischen Implantaten die Oberflächenstruktur dem Knochen dadurch angepaßt, daß der massive Kern des Implantates von einer offenporigen, schwammähnlichen Struktur bedeckt ist, deren Verbindungsstege sich zum Kern hin verdichten. Dadurch sind der Körperflüssigkeit ideale Möglichkeiten gegeben, die Grenze zwischen Implantat und Humangewebe zu durchströmen und mit Nährstoffen zu versorgen und so das Verwachsen zu fördern.

Auf der anderen Seite wird die Oberflächemorphologie von Implantaten erfolgreich mit Hilfe der Beschichtungstechnik beeinflußt. So ist z.B. bekannt, daß Implantate, mit einer Ti-Spritzschicht überzogen, unmittelbar an der Oberfläche mit dem Knochen verwachsen und so ohne Zement Stabilität erlangen. Diese Verwachsung geschieht umso besser, je poröser die Spritzschicht, je rauher ihre Oberfläche und je "bioaktiver" der Beschichtungswerkstoff ist. $TiO_2$ z.B. regt das Knochenwachstum so an, daß sich keine schwieligen Bindegewebeschichten ausbilden.

Weiter wird heute versucht, mit Materialien der Apatit-Stoffgruppe, beispielsweise Hydroxylapatit, das Einwachsen des Implantates dadurch zu beschleunigen, daß es aufgrund ihrer physiologischen Ähnlichkeit zur Knochensubstanz zu Austauschvorgängen kommt. Allerdings steht dem Vorteil des raschen und zunächst stabilen Einwachsens des Implantates der Nachteil der Lockerungsgefahr zu einem späteren Zeitpunkt gegenüber, verursacht durch ein Auflösen der Apatitschicht oder ihre Umwandlung in eine labile Reaktionszone.

Der Erfindung liegt die Aufgabe zugrunde, einen Implantatkörper der eingangs beschriebenen Art zu schaffen, welcher nicht nur ideale Einwachsbedingungen in den ungebundenen Knochen erzeugt, sondern auch einen dauerhaften Halt im knöchernen Gewebe trotz ständiger mechanischer Belastung sicherstellt. Weiter soll die Beschichtung auf allen Implantaten zu identischer Oberflächenmorphologie führen, welche vollständig unabhängig vom Trägermaterial ist, so daß die eigentliche Prothesenmasse nur nach Stabilitätsgesichtspunkten ausgewählt zu werden braucht und selbst keine besondere Gewebefreundlichkeit besitzen muß.

Diese Aufgabe wird erfindungsgemäß durch einen Implantatkörper mit dem im Anspruch 1 angegebenen Merkmalen gelöst. Dabei wird auf den Implantatkörper aus beliebigem Material zunächst eine Titangrundsicht aufgebracht, welche aus einer ersten sehr dichten Lage und einer zweiten sehr rauhen Lage mit gezielt eingestellter Großporigkeit besteht, und auf die Titangrundsicht folgt eine Zwischenschicht und auf diese eine dünne, sehr dichte Deckschicht aus Bioaktiv-Material. Die Zwischenschicht besteht aus einer Mischung der Materialien der Titangrundschicht und der Deckschicht.

Zwischenschicht und Deckschicht sind derart ausgebildet, daß die Rauhigkeit und Großporigkeit der zweiten Lage der Titangrundschicht auf der Oberfläche der Deckschicht erhalten bleibt.

Die Schichten sind vorteilhaft durch Plasmaspritzen, bevorzugt durch Vakuumplasmaspritzen (VPS) aufgebracht. Die Ausführung der Beschichtung in der VPS-Technik erfolgt aus Gründen der Hygiene, aber auch aus Gründen der ansich bekannten verbesserten Abscheidebedingungen und Schichteigenschaften.

Für sehr kleine Implantatkörper wird die Körnung des Spritzpulvers für die Titangrundschicht zweckmäßig auf etwa 60 μm begrenzt.

Für die Beschichtung größerer Implantatkörper beträgt die Körnung des Spritzpulvers für die Titangrundschicht vorteilhaft höchstens etwa 100 bis etwa 250 μm.

Zweckmäßig enthält die erste Lage der Titangrundschicht nur aufgeschmolzene Spritzpulverpartikel, während deren zweite Lage auch unaufgeschmolzene Spritzpulverpartikel enthält.

Die Zwischenschicht kann vorteilhaft einen gradierten Übergang vom Material der Titangrundschicht zum Material der Deckschicht aufweisen.

Besonders vorteilhaft ist das Material der Deckschicht $TiO_2$. Die Zwischenschicht besteht dann aus einer Mischung aus Ti und $TiO_2$, während die Deckschicht eine dichtgespritzte, reine $TiO_2$-Schicht darstellt.

Das Bioaktiv-Material der Deckschicht kann ein Material aus der Apatit-Stoffgruppe, vorzugsweise Hydroxylapatit sein.

Vorteilhaft ist die Deckschicht etwa 50 μm dick, während Zwischenschicht und Deckschicht zusammen etwa 200 μm dick sind.

Die Oberfläche des Implantatkörpers ist zweckmäßig vor dem Aufbringen der Beschichtung vorzugsweise mit $Al_2O_3$ sandgestrahlt und damit aufgerauht.

Im Falle von metallischen Implantmaterialien ist die Oberfläche des Implantatkörpers vorteilhaft unmittelbar vor dem Aufbringen der Beschichtung in einem Sputterprozeß mit Hilfe des übertragenen Lichtbogens getrocknet und von absorbierten Gasen und dünnen Oxidhäuten befreit.

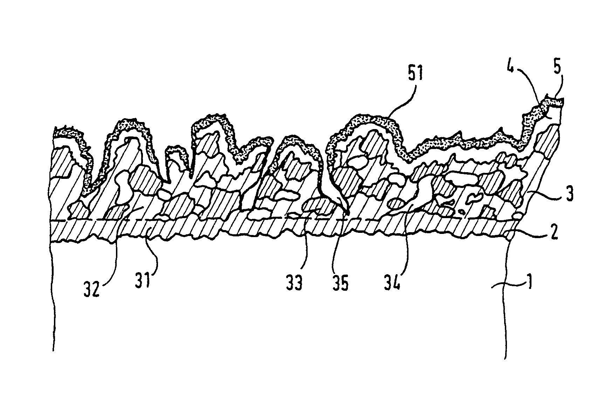

Die Erfindung ist im folgenden anhand der Zeichnung näher erläutert.

Die einzige Figur der Zeichnung zeigt einen Schnitt durch eine erfindungsgemäße Implantatbeschichtung.

In der Zeichnung ist ein Implantatkörper 1 dargestellt, dessen Oberfläche 2 durch Sandstrahlen, vorzugsweise mit $Al_2O_3$-Partikeln, aufgerauht sein kann und im Falle von metallischen Implantatmaterialien durch einen Sputterprozeß mit Hilfe des übertragenen Lichtbogens unmittelbar vor dem Beschichten mit einer Titangrundschicht 3 getrocknet und von adsorbierten Gasen und dünnen Oxidhäuten befreit wurde. Auf diese Weise ist eine ausreichende Haftkraft der Beschichtung gewährleistet und gleichzeitig eine erste Sterilisation durch die Inertgasplasmaflamme erreicht.

Die Titangrundschicht 3 wird nun zunächst mit einer solchen Plasmaflammenergie abgeschieden, daß sämtliche Ti-Spritzpulverpartikel aufschmelzen und eine sehr dichte erste Lage 31 entsteht. Schon nach wenigen Schichtlagen kann dann die Flammenergie so abgesenkt werden, daß nur noch die Feinpartikelanteile des Ti-Spritzpulvers aufgeschmolzen werden, gröbere Spritzpulverpartikel 33 aber nicht mehr, so daß eine großporige sehr rauhe zweite Lage 32 der Titangrundschicht 3 mit großen Poren 34 und Spalten 35 entsteht. Dabei sind die nicht geschmolzenen Spritzpulverpartikel 33 durch die geschmolzenen untereinander stabil verbunden.

Je nach Implantat kann die Großporigkeit und Rauhigkeit der Titangrundschicht 3 durch die Kornfraktionierung und durch die gewählte Flammenergie eingestellt werden. Für sehr kleine Implantate ist eine Begrenzung der Körnung bei etwa 60 μm vorzunehmen, während bei der Beschichtung von größeren Implantaten eine wesentlich stärkere Aufrauhung und größere Porendurchmesser durch Ausweitung der Spritzpulverkörnung auf 100 bis 250 μm empfehlenswert sind, so daß die Knochensubstanz in die so erzeugten Poren und Nischen einwachsen kann.

Auf die Titangrundschicht 3 wird nun eine Zwischenschicht 4 aufgebracht, welche aus einer Mischung von Bioaktiv-Material, beispielsweise Hydroxylapatit, und Titan besteht und im wesentlichen als dünne Haftschicht für eine nachfolgende Deckschicht 5 aus reinem Bioaktiv-Material dient. Dabei sind Zwischenschicht 4 und Deckschicht 5 so aufgetragen, daß die gezielt eingestellte Großporigkeit und Rauhigkeit der Titangrundschicht 3 auf der Oberfläche 51 der Deckschicht 5 erhalten bleiben. Ihre Oberflächenmorphologie hat sich durch die weitere Beschichtung hindurch an die neu gebildete Oberfläche 51 aus Bioaktiv-Material übertragen.

Wichtig ist die geringe Schichtdicke des Bioaktiv-Materials. Zwischenschicht 4 und Deckschicht 5 zusammen sind nicht dicker als 200 μm, wobei die geschlossene Deckschicht 5 etwa 50 μm dick ist. Ihre Aufgabe besteht letztlich nur darin, ein rasches Verwachsen auszulösen. Mit Hilfe der Zwischenschicht 4 ist sie haftfest mit der Titangrundschicht 3 verbunden.

Hauptvorteil einer solchen erfindungsgemäß hergestellten Implantatbeschichtung ist die rasche und stabile Verwachsung des Bioaktivstoffes mit der Knochensubstanz. Die eingestellte Großporigkeit und Rauhigkeit der Oberfläche gibt zusätzliche Stabilität und sichert die Versorgung mit Nährstoffen durch die Zirkulierbarkeit der Körperflüssigkeiten. Vor allem entsteht mit höchster Währscheinlichkeit eine sichere Langzeitstabilität, da im Falle einer Auflösung des Bioaktiv-Materials die Titanoberfläche mit der im Vakuumplasmaspritzverfahren optimal dünnen $TiO_2$-Oberflächenpassivierung als Reaktionspartner des Humangewebes in Erscheinung tritt. Durch die Ausbildung der Zwischenschicht 4 als Mischung aus Titan und Bioaktiv-Material wird mit zunehmender Auflösung die Knochensubstanz geradezu gezwungen, sich mit der TiO$_2$-Oberfläche zu verbinden.

Als weiterer Vorteil der erfindungsgemäß hergestellten Implantatbeschichtung ist die Vergrößerung der Oberfläche des Implantates zu nennen. Deshalb kann die Oberfläche des Trägerkörpers glatt sein und ist damit einfach herstellbar.

Aufgrund der gewählten Schichtwerkstoffe kann die beschichtete Prothese beliebig oft und sicher sterilisiert werden.

**Patentansprüche**

1. Implantatkörper mit poriger, biokompatibler Beschichtung mit folgenden Merkmalen:
der Implantatkörper (1) ist aus beliebigem Material;
auf dem Implantatkörper (1) ist eine Titangrundschicht (3) der beschichtung aufgebracht;
die Titangrundschicht (3) besteht aus einer ersten sehr dichten Lage (31) und einer zweiten sehr rauhen Lage (32) mit gezielt eingestellter Großporigkeit aufgrund von Poren (34) und Spalten (35);
auf die Titangrundschicht (3) ist eine Zwischenschicht (4) und auf diese eine dünne, sehr dichte Deckschicht (5) aus Bioaktiv-Material aufgebracht;
die Zwischenschicht (4) besteht aus einer Mischung der Materialien der Titangrundschicht (3) und der Deckschicht (5);
die Zwischenschicht (4) und die Deckschicht (5) sind derart ausgebildet, daß die Rauhigkeit und Großporigkeit der zweiten Lage (32) der Titangrundschicht auf der Oberfläche (51) der Deckschicht (5) erhalten bleibt.

2. Implantatkörper nach Anspruch 1, dadurch gekennzeichnet, daß die Schichten (3, 4, 5) durch Plasmaspritzen aufgebracht sind.

3. Implantatkörper nach Anspruch 1 oder 2, dadurch gekennzeichnet, daß die Schichten (3, 4, 5) durch Vakuumplasmaspritzen aufgebracht sind.

4. Implantatkörper nach Anspruch 2 oder 3, dadurch gekennzeichnet, daß die Körnung des Spritzpulvers für die Titangrundschicht (3) höchstens etwa 60 µm beträgt.

5. Implantatkörper nach Anspruch 2 oder 3, dadurch gekennzeichnet, daß die Körnung das Spritzpulvers für die Titangrundschicht (3) höchstens etwa 100 bis etwa 250 µm beträgt.

6. Implantatkörper nach einem der Ansprüche 2 bis 5, dadurch gekennzeichnet, daß die erste Lage (31) zur aufgeschmolzene Spritzpulverpartikel und die zweite Lage (32) auch unaufgeschmolzene Spritzpulverpartikel (33) enthält.

7. Implantatkörper nach einem der vorhergehenden Ansprüche, dadurch gekennzeichnet, daß die Zwischenschicht (4) einen gradierten Übergang vom Material der Titangrundschicht (3) zum Material der Deckschicht (5) aufweist.

8. Implantatkörper nach einem der vorhergehenden Ansprüche, dadurch gekennzeichnet, daß das Material der Deckschicht (5) TiO$_2$ ist.

9. Implantatkörper nach einem der Ansprüche 1 bis 7, dadurch gekennzeichnet, daß das Material der Deckschicht (5) ein Material aus der Apatit-Stoffgruppe ist.

10. Implantatkörper nach Anspruch 9, dadurch gekennzeichnet, daß das Material der Deckschicht (5) Hydroxylapatit ist.

11. Implantatkörper nach einem der vorhergehenden Ansprüche, dadurch gekennzeichnet, daß Deckschicht (5) etwa 50 µm dick und Zwischenschicht (4) und Deckschicht (5) zusammen etwa 200 µm dick sind.

12. Implantatkörper nach einem der vorhergehenden Ansprüche, dadurch gekennzeichnet, daß die Oberfläche (2) des Implantatkörpers (1) vor dem Aufbringen der Beschichtung (3, 4, 5) mit Al$_2$O$_3$ sandgestrahlt ist.

13. Implantatkörper nach einem der Ansprüche 2 bis 12, dadurch gekennzeichnet, daß im Falle von metallischen Implantatmaterialien die Oberfläche (2) des Implantatkörpers (1) unmittelbar vor dem Aufbringen der Beschichtung (3, 4, 5) in einem Sputterprozeß mit Hilfe des übertragenen Lichtbogens getrocknet und von absorbierten Gasen und dünnen Oxidhäuten befreit ist.

**Revendications**

1. Corps d'implant portant un revêtement poreux, biocompatible, ayant les caractéristiques suivantes:
le corps d'implant (1) est fait d'un matériau choisi librement;
une couche de base de titane (3) du revêtement est appliquée sur le corps d'implant (1);
la couche de base de titane (3) est constituée d'une première sous-couche (31) très dense et d'une deuxième sous-couche (32) très rugueuse, ayant une macroporosité ajustée de façon adéquate et qui est due à des pores (34) et des crevasses (35);
une couche intermédiaire (4) est appliquée sur la couche de base de titane (3) et une mince couche de recouvrement (5) très dense, en matériau bioactif, est appliquée sur la couche intermédiaire;
la couche intermédiaire (4) est formée d'un mélange des matériaux de la couche de base de titane (3) et de la couche de recouvrement (5); et
la couche intermédiaire (4) et la couche de recouvrement (5) sont conformées de manière que la rugosité et la macroporosité de la deuxième sous-couche (32) de la couche de base de titane soient conservées à la surface (51) de la couche de recouvrement (5).

2. Corps d'implant selon la revendication 1, caractérisé en ce que les couches (3, 4, 5) sont appliquées par projection au plasma.

3. Corps d'implant selon la revendication 1 ou 2, caractérisé en ce que les couches (3, 4, 5) sont appliquées par projection au plasma dans le vide.

4. Corps d'implant selon la revendication 2 ou 3, caractérisé en ce que la grosseur de grain de la poudre à projeter pour la couche de base de titane (3) est tout au plus d'environ 60 µm.

5. Corps d'implant selon la revendication 2 ou 3, caractérisé en ce que la grosseur de grain de la poudre à projeter pour la couche de base de titane (3) est tout au plus d'environ 100 à environ 250 µm.

6. Corps d'implant selon une des revendications 2 à 5, caractérisé en ce que la première sous-couche (31) contient seulement des particules fondues de la poudre projetée et la deuxième sous-couche (32) contient aussi des particules (33) non fondues de la poudre projetée.

7. Corps d'implant selon une des revendications précédentes, caractérisé en ce que la couche intermédiaire (4) présente une transition graduée du matériau de la couche de base de titane (3) au matériau de la couche de recouvrement (5).

8. Corps d'implant selon une des revendications précédentes, caractérisé en ce que le matériau de la couche de recouvrement (5) est du TiO₂.

9. Corps d'implant selon une des revendications 1 à 7, caractérisé en ce que le matériau de la couche de recouvrement (5) est un matériau du groupe des substances à base d'apatite.

10. Corps d'implant selon la revendication 9, caractérisé en ce que le matériau de la couche de recouvrement (5) est l'hydroxylapatite.

11. Corps d'implant selon une des revendications précédentes, caractérisé en ce que la couche de recouvrement (5) possède une épaisseur d'environ 50 µm et l'épaisseur globale de la couche intermédiaire (4) et de la couche de recouvrement (5) est d'environ 200 µm.

12. Corps d'implant selon une des revendications précédentes, caractérisé en ce que la surface (2) du corps d'implant (1) est sablée avec du Al₂O₃ avant l'application du revêtement (3, 4, 5).

13. Corps d'implant selon une des revendications 2 à 12, caractérisé en ce que, en cas d'utilisation de matériaux d'implants métalliques, la surface (2) du corps d'implant (1) est soumise, immédiatement avant l'application du revêtement (3, 4, 5), dans un processus de pulvérisation cathodique, à l'aide de l'arc transféré, à un séchage et à l'élimination de gaz absorbés et de minces peaux d'oxyde.

## Claims

1. Implant body with porous, biologically compatible coating with the following features:
the implant body (1) is made from any desired material;
to the implant body (1) is applied a titanium base layer (3) of the coating;
the titanium base layer (3) consists of a very dense first layer (31) and a very rough second layer (32) with the pores (34) and cracks (35) deliberately adjusted to be large;
to the titanium base layer (3) is applied an intermediate layer (4) and to this is applied a thin, very dense coating layer (5) of biologically active material;
the intermediate layer (4) consists of a mixture of the materials from the titanium base layer (3) and the coating layer (5);
the intermediate layer (4) and the coating layer (5) are constructed in such a way that the roughness and large pore size of the second layer (32) of the titanium base layer is maintained on the surface (51) of the coating layer (5).

2. Implant body according to claim 1, characterised in that the layers (3, 4, 5) are applied by plasma spraying.

3. Implant body according to claim 1 or 2, characterised in that the layers (3, 4, 5) are applied by vacuum plasma spraying.

4. Implant body according to claim 2 or 3, characterised in that the particle size of the spray powder for the titanium base layer (3) is about 60 µm at the most.

5. Implant according to claims 2 or 3, characterised in that the particle size of the spray powder for the titanium base layer (3) is between about 100 to 250 µm at the most.

6. Implant body according to one of the claims 2 to 5, characterised in that the first layer (31) contains only molten spray powder particles and the second layer (32) also contains non-molten spray powder particles (33).

7. Implant body according to one of the preceding claims, characterised in that the intermediate layer (4) has a gradual transition from the material of the titanium base layer (3) to the material of the coating layer (5).

8. Implant body according to one of the preceding claims, characterised in that the material of the coating layer (5) is TiO₂.

9. Implant body according to one of the claims 1 to 7, characterised in that the material of the coating layer (5) is a material from the apatite group of substances.

10. Implant body according to claim 9, characterised in that the material of the coating layer (5) is hydroxylapatite.

11. Implant body according to one of the preceding claims, characterised in that the coating layer (5) is about 50 µm thick and the intermediate layer (4) and the coating layer (5) together are about 200 µm thick.

12. Implant body according to one of the preceding claims, characterised in that the surface (2) of the implant body (1) is sand blasted with Al₂O₃ before the application of the coating (3, 4, 5).

13. Implant body according to one of the claims 2 to 12, characterised in that in the case of metallic implant materials the surface (2) of the implant body (1) is dried immediately before the application of the coating (3, 4, 5) in a sputter process with help of the transferred electric arc and is freed from absorbed gases and thin oxide films.